# EUROPEAN PATENT SPECIFICATION

(11) **EP 1 146 873 B1**
(45) Date of publication and mention of the grant of the patent: **16.03.2005**
(21) Application number: 00905748.0
(22) Date of filing: 25.01.2000
(51) Int. Cl.: A61K 31/435, A61K 31/47, A61P 5/28

(54) **ANTI-ANDROGENS AND METHODS FOR TREATING DISEASE**
ANTI-ANDROGENE UND VERFAHREN ZUR BEHANDLUNG VON KRANKHEITEN
ANTI-ANDROGENES ET PROCEDES DE TRAITEMENT

(30) Priority: 25.01.1999 US 117059 P
(43) Date of publication of application: 24.10.2001
(73) Proprietor: SMITHKLINE BEECHAM CORPORATION, King of Prussia, PA 19406-0939 (US)
(72) Inventor: MURPHY, Dennis, Chester Springs, PA 19425 (US); WIER, Patrick J., King of Prussia, PA 19406-0939 (US); GIARDINA, Giuseppe Arnaldo Maria, I-20021 Baranzate (IT)
(74) Representative: Rutter, Keith
(86) International application number: PCT/US2000/001956
(87) International publication number: WO 2000/043008

(56) References cited:
- EP-A2- 0 940 391
- WO-A-93/23053
- WO-A-97/04002
- WO-A1-96/02509
- US-A- 5 120 840
- US-A- 5 380 728
- US-A- 5 418 238
- PRITCHARD MARTYN C ET AL: "Tachykinin NK-3 receptors: Biology and development of selective peptide and nonpeptide ligands." DRUGS OF THE FUTURE, vol. 20, no. 11, 1995, pages 1163-1173, XP008012878 ISSN: 0377-8282

## Description

### FIELD OF THE INVENTION:

This invention relates to a method of treatment and/or prophylaxis of diseases, in a mammal, for which gonadotropin suppression and/or androgen suppression is desired. Gonadotropin suppression is achieved according to this invention by administration of a neurokinin-3 (NK-3) receptor antagonist. More in particular, this invention relates to a method of gonadotropin suppression, e.g., by lowering luteinizing hormone (LH) blood levels by administering to a mammal in need thereof, an effective amount of an NK-3 receptor antagonist. Concurrently or alternatively with gonadotropin suppression, this invention also relates to suppression of androgen production with NK-3 receptor antagonists. Suitable NK-3 receptor antagonists useful in this invention are represented by the compounds described herein.

### BACKGROUND OF THE INVENTION:

Gonadotropins, including LH and follicle-stimulating hormone (FSH), are released by the pituitary gland and stimulate the production of steroid hormones by the gonads. Androgens, the principal one being testosterone, are secreted mainly by the testes and, to a lesser degree, by the adrenal cortex and ovary. In males, they are involved in the development and maintenance of secondary sexual characteristics and several physiological functions, such as normal reproduction, sexual performance ability, and skeleton and skeletal muscle growth and development during puberty. In females, they stimulate growth of pubic hair and probably libido, as well as acting as estrogen precursors. Antiandrogens are drugs that antagonize the effects of androgens. Antiandrogens are known to be useful in treating benign prostatic hyperplasia (BPH), androgen-dependent acne, and prostatic cancer. Korolkovas, A., Essentials Of Medicinal Chemistry, Second Edition, pp. 1009-1015 (1988).

Steroid hormones, including testosterone, not only play an important role in regulating growth, differentiation and function of organs such as the gonads, pituitary and sex organs, but, they are also involved in mediating BPH, and many cancers, including those of the prostate, e.g., metastatic prostatic carcinoma, breast, testicles and endometrium. H. Vanden Bossche, *J. Steroid Biochem. Molec. Biol.,* Vol. 43, No. 8, pp. 1003-1021 (1992).

BPH is a disease in which the prostate resumes growth late in life.. Hormonal imbalance plays an important role among the etiologic factors of BPH (Sciarra et al., Antiandrogens: clinical applications, *J. Steroid Biochem. Molec. Biol.,* Vol. 37, pp. 349-362 (1990)). The impact of androgens on prostatic carcinoma is known, as is the treatment of prostate cancer by androgen deprivation, including androgen blockade and inhibition of androgen synthesis (Huggins et al., *Archs. Surg.,* Vol. 43, pp. 209-223 (1941)).

In addition, steroid hormones are widely used as contraceptives. Antispermatogenic agents are male contraceptives that inhibit spermatogenesis, the process leading to mature spermatazoa. Drugs that interfere in this process include androgens and antiandrogens. Since the antiandrogenic effects of the compounds claimed herein are reversible, the compounds may be useful as a male contraceptive agent. Korolkovas, A., Essentials Of Medicinal Chemistry, Second Edition, pp. 1032 (1988).

Hyperandrogenism (excess production and secretion of androgens and precursors) is a common and sometimes serious endrocrinopathy for women of reproductive age. The excess androgens and precursors originate from the adrenal glands and ovaries in various proportions and manifest in varying effects depending on the amount of excess androgen. Clinical manifestations range from hirsutism (excessive hair growth of male pattern, sometimes accompanied by acne) to virilization (clitorimegaly, temporal balding, deepening of voice, or enhanced musculature). Hyperandrogenism occurs as part of a wide spectrum of disease manifestations, including polycystic ovary syndrome (PCOS) which is a variable combination of hirsutism, infertility, obesity, insulin resistance and polycystic ovaries, the HAIR-AN syndrome (hyperandrogenism, insulin resistance and acanthosis nigricans), ovarian hyperthecosis (HAIR-AN with hyperplasia of luteinized theca cells in ovarian stroma), and other manifestations of high intraovarian androgen concentrations (e.g., follicular maturation arrest, atresia, anovulation, dysmenorrhea, dysfunctional uterine bleeding, infertility), androgen-producing tumors (virilizing ovarian or adrenal tumors).

Current medical therapies for women are directed against the adrenals, the ovaries or the androgen receptor. Glucocorticoid therapy is directed against the adrenal glands but is limited, in some cases, by unwanted suppression of cortisol synthesis. GnRH therapy is directed against the ovaries, but is expensive, and its long-term effects are unknown. Further, therapy using oral contraceptives may be unsuitable because most contain progestins with androgenic activity.

Because the abnormal production of androgens is implicated in the pathways of many diseases and/or disorders for which there are no acceptable treatments, a need exists to find small molecules to inhibit the production of gonadotropins and/or androgens in mammals for their treatment and/or prophylaxis. The current invention teaches that NK-3 receptor antagonists broadly, and the compounds described herein specifically, suppress gonadotropin production and/or androgen production/secretion, and therefore, are useful in the treatment and/or prophylaxis of diseases or disorders in which decreased levels of androgens are desired.

In men these diseases or disorders, include, but are not limited to, BPH, prostatic hyperplasia, metastatic prostatic carcinoma, testicular cancer, androgen dependent acne, male pattern baldness and precocious puberty in boys. In addition, according to this invention, NK-3 receptor antagonists and the compounds herein would be useful as male contraceptives and to inhibit the sex drive in male sexual offenders.

In women, the above-mentioned syndromes manifest from excess androgen production/secretion. Therefore, administration of an NK-3 receptor antagonist according to this invention would be an effective treatment of those syndromes. In addition, administration of an NK-3 receptor antagonist according to this invention would be useful in the suppression of preovulatory LH surges in assisted reproductive technologies, and in treating or preventing endometriosis, estrogen-dependent (or positive) breast cancers, and uterine leiomyomas (fibroids).

### SUMMARY OF THE INVENTION:

The present invention is to the novel use of antagonists of the NK-3 receptor to achieve gonadotropin and/or androgen suppression in mammals. According to this invention, NK-3 receptor antagonists are administered for the treatment and/or prophylaxis of diseases which are caused by abnormal levels of androgens, particularly testosterone. The compounds described herein by reference are particularly useful according to this invention.

### DETAILED DESCRIPTION OF THE INVENTION:

Surprisingly, it has now been found that NK-3 receptor antagonists are effective in reversibly suppressing gonadotropin and/or androgen production/secretion, specifically the production of LH and/or testosterone in mammals. Suppression of gonadotropin production/secretion results in the suppression of androgen production/secretion. Thus, the instant invention is in a method of treatment and/or prophylaxis of disease states or disorders which are effected, or exacerbated by, elevated and/or abnormal levels of gonadotropins and/or androgens. Specifically, the invention relates to a method of suppressing gonadotropin and/or androgen blood levels by administering an effective amount of an NK-3 receptor antagonist. The compounds defined herein exhibit the ability to suppress gonadotropin and/or androgen production, and in particular, are effective in reversibly lowering blood levels of LH and the androgen testosterone.

The term "androgen" is used herein to mean steroids that encourage the development of male sex characteristics and include the steroid derivatives of androstane including, testosterone, androstenedione, and analogs.

As used herein, a disease state or disorder characterized by "androgenic dependency" is a disease state which is exacerbated by, or caused by, insufficient, excessive, inappropriate or unregulated androgen production. Examples of such diseases in men include, but are not limited to, BPH, metastatic prostatic carcinoma, testicular cancer, androgen dependent acne, male pattern baldness and precocious puberty in boys. Examples of such diseases in women include, hyperandrogenism, hirsutism, virilization, POCS, HAIR-AN syndrome, ovarian hyperthecosis, follicular maturation arrest, atresia, anovulation, dysmenorrhea, dysfunctional uterine bleeding, infertility, androgen-producing tumors.

As used herein, "gonadotropin suppression" refers to the reduction in the production or synthesis of one or more naturally occurring gonadotropins, including lutinizing hormone and follicle-stimulating hormone.

As used herein, "androgen inhibiting" refers to an effective amount of an NK-3 receptor antagonist or other compound defined herein, which will cause a decrease in the *in vivo* levels of the androgen to normal or sub-normal levels, when given to a patient for the prophylaxis or treatment of a disease state which is exacerbated by, or caused by, excessive or unregulated androgen production. Without being bound to a particular theory of this invention, current findings indicate that the antiandrogenic activity of the NK-3 receptor antagonists described herein involves a direct inhibition of testicular synthesis of testosterone and/or a suppression of LH release from the pituitary.

Compounds for use herein include the NK-3 receptor antagonists as described in, and made according to, WO 95/32948, published December 7, 1995, as Farina et al.; WO 96/02509, published February 1, 1996, as Farina et al.; WO 97/19926, published June 5, 1997, as Giardina et al.; WO 97/21680, published June 19, 1997, as Giardina et al.; WO 98/52942, published November 26, 1998, as Giardina et al., WO 98/05640, published February 12, 1998, as Grugni et al., and EP0673928, published September 27, 1995, as Bichon et al.

Particularly preferred compounds for use herein are as follows:
((S)-(-)-N-(α-Ethylbenzyl)-3-hydroxy-2-phenylquinoline-4-carboxamide);
(S)-(-)-N-(α-Ethylbenzyl)-3-[3-[4-(2-methoxyphenyl)piperazin-1-yl]propoxy]-2-phenylquinoline-4-carboxamide;
(+)-(R)-3-[3-(4-Phenyl-4-N-methylacetamidopiperidin-1-yl)propyl]-3-(3,4-dichlorophenyl)-1-benzoylpiperidine;
(S)-(-)-N-(α-Ethylbenzyl)-3-(3-carboxy)propoxy-2-phenylquinoline-4-carboxamide; and
(S)-(-)-N-(α-Ethylbenzyl)-3-carboxymethoxy-2-phenylquinoline-4-carboxamide.

Methods for preparing the compounds described above as useful in this invention are described in WO 95/32948, published December 7, 1995, as Farina et al.; WO 96/02509, published February 1, 1996, as Farina et al.; WO 97/19926, published June 5, 1997, as Giardina et al.; WO 97/21680, published June 19, 1997, as Giardina et al.; WO 98/52942, published November 26, 1998, as Giardina et al., and WO 98/05640, published February 12, 1998, as Grugni et al.

The present invention further provides a pharmaceutical composition comprising an NK-3 receptor antagonist or a compound described above, or a pharmaceutically acceptable salt or solvate thereof, and a pharmaceutically acceptable carrier.

The present invention also provides the use of an NK-3 receptor antagonist or a compound as described herein, or a pharmaceutically acceptable salt or solvate thereof, in the manufacture of a medicament for the treatment and/or prophylaxis of diseases in which modified levels of androgens are desired, including, but not limited to, BPH, metastatic prostatic carcinoma, testicular cancer, androgen dependent acne, male pattern baldness, precocious puberty in boys, hyperandrogenism, hirsutism, virilization, POCS, HAIR-AN syndrome, ovarian hyperthecosis, follicular maturation arrest, atresia, anovulation, dysmenorrhea, dysfunctional uterine bleeding, infertility, androgen-producing tumors.

Such a medicament, and a composition of this invention, may be prepared by admixture of a compound of the invention with an appropriate carrier. It may contain a diluent, binder, filler, disintegrant, flavoring agent, coloring agent, lubricant or preservative in conventional manner.

These conventional excipients may be employed for example as in the preparation of compositions of known agents for treating the conditions.

Preferably, a pharmaceutical composition of the invention is in unit dosage form and in a form adapted for use in the medical or veterinarial fields. For example, such preparations may be in a pack form accompanied by written or printed instructions for use as an agent in the treatment of the conditions.

The suitable dosage range for the compounds of the invention depends on the compound to be employed and on the condition of the patient. It will also depend, inter alia, upon the relation of potency to absorbability and the frequency and route of administration.

The compound or composition of the invention may be formulated for administration by any route, and is preferably in unit dosage form or in a form that a human patient may administer to himself in a single dosage. Advantageously, the composition is suitable for oral, rectal, topical, parenteral, intravenous or intramuscular administration. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinyl-pyrrolidone, sodium starch glycollate or microcrystalline cellulose; or pharmaceutically acceptable setting agents such as sodium lauryl sulphate.

Solid compositions may be obtained by conventional methods of blending, filling, tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminum stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavoring or coloring agents.

The compounds of this invention may also be administered by a non-oral route. In accordance with routine pharmaceutical procedure, the compositions may be formulated, for example for rectal administration as a suppository. They may also be formulated for presentation in an injectable form in an aqueous or non-aqueous solution, suspension or emulsion in a pharmaceutically acceptable liquid, e.g. sterile pyrogen-free water or a parenterally acceptable oil or a mixture of liquids. The liquid may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

The compounds of this invention may also be administered by inhalation, via the nasal or oral routes. Such administration can be carried out with a spray formulation comprising a compound of the invention and a suitable carrier, optionally suspended in, for example, a hydrocarbon propellant.

Preferred spray formulations comprise micronised compound particles in combination with a surfactant, solvent or a dispersing agent to prevent the sedimentation of suspended particles. Preferably, the compound particle size is from about 2 to 10 µm.

A further mode of administration of the compounds of the invention comprises transdermal delivery utilizing a skin-patch formulation. A preferred formulation comprises a compound of the invention dispersed in a pressure sensitive adhesive which adheres to the skin, thereby permitting the compound to diffuse from the adhesive through the skin for delivery to the patient. For a constant rate of percutaneous absorption, pressure sensitive adhesives known in the art such as natural rubber or silicone can be used.

As mentioned above, the effective dose of compound depends on the particular compound employed, the condition of the patient and on the frequency and route of administration. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

No unacceptable toxicological effects are expected with compounds of the invention when administered in accordance with the invention.

### BIOLOGICAL DATA:

The antiandrogenic effects of the compounds useful herein are determined by the following summary which demonstrates reproductive organ and testosterone effects.

Once daily oral administration of ((S)-(-)-N-(α-Ethylbenzyl)-3-hydroxy-2-phenylquinoline-4-carboxamide) for 1 month to sexually mature male beagle dogs (12 - 16 months of age) produced decreases in the size and/or weight of testes and prostate. Histologic evaluation of these tissues revealed degeneration of the seminiferous epithelium of the testes with depletion of spermatogonia, spermatocytes and spermatids and diffuse atrophy of the prostate gland. The incidence and severity of these changes were dose-related with 1 of 3, 2 of 3, 2 of 3, 6 of 6 and 3 of 3 male dogs affected at doses of 0.3, 3, 30, 300 or 1000 mg/kg per day, respectively. The reversibility of these changes has also been demonstrated.

Once daily oral administration of ((S)-(-)-N-(α-Ethylbenzyl)-3-hydroxy-2-phenylquinoline-4-carboxamide) for 1 month to male rats or for 2 weeks to male mice had no effect on the weights and/or histologic appearance of the reproductive organs. The daily doses ranged from 0.1 to 1000 mg/kg per day.

The ((S)-(-)-N-(α-Ethylbenzyl)-3-hydroxy-2-phenylquinoline-4-carboxamide)-dependent changes in the testes and prostate of male dogs were associated with reductions in serum concentration of testosterone. Testosterone concentrations were reduced after either a single oral dose of 300 mg/kg (3 of 3 dogs) or 25 daily oral doses of 0.3 (1 of 3 dogs), 3 (2 of 3 dogs) or 300 mg/kg ((S)-(-)-N-(α-Ethylbenzyl)-3-hydroxy-2-phenylquinoline-4-carboxamide) (3 of 3 dogs). Serum testosterone was measured on blood samples obtained every 2 hours for 24 hours after the single dose and every 20 minutes for 4 hours beginning approximately 2 hours after the last of the 25 daily doses. The reductions in serum testosterone observed after one or 25 daily doses of 300 mg/kg ((S)-(-)-N-(α-Ethylbenzyl)-3-hydroxy-2-phenylquinoline-4-carboxamide) were reversible in all dogs (6 of 6) within one week after the cessation of dosing.

The growth and maintenance of the testes and prostate are androgen dependent. Consequently, the testicular degeneration and prostatic atrophy observed in dogs given ((S)-(-)-N-(α-Ethylbenzyl)-3-hydroxy-2-phenylquinoline-4-carboxamide) appears to be associated with the reduced serum concentration of testosterone. Furthermore, because testosterone is produced in the testes (Leydig cells), the occurrence of testicular degeneration indicates that the reduction in serum testosterone is due to an inhibition of synthesis, and not to an enhanced elimination of circulating testosterone.

A single oral dose of 300 mg/kg (S)-(-)-N-(α-Ethylbenzyl)-3-hydroxy-2-phenylquinoline-4-carboxamide produced a rapid and marked decrease in serum concentration of testosterone in male dogs that lasted for at least 24 hours post-dose. This effect was reversible and was associated with a reduction in serum concentration of luteinizing hormone (LH). This dose of (S)-(-)-N-(α-Ethylbenzyl)-3-hydroxy-2-phenylquinoline-4-carboxamide also reduced the plasma concentration of androstenedione, but had no effect on plasma concentrations of estradiol or cortisol in male dogs. A single intravenous dose of 10 mg/kg (approximately 26 umoles/kg) (S)-(-)-N-(α-Ethylbenzyl)-3-hydroxy-2-phenylquinoline-4-carboxamide also reduced plasma concentration of testosterone in male dogs. The quinoline derivatives (S)-(-)-N-(α-Ethylbenzyl)-3-[3-[4-(2-methoxyphenyl)piperazin-1-yl]propoxy]-2-phenylquinoline-4-carboxamide and (S)-(-)-N-(α-Ethylbenzyl)-3-carboxymethoxy-2-phenylquinoline-4-carboxamide, which possess NK-3 receptor antagonist activity, reduced plasma concentration of testosterone in male dogs when given intravenously at a dose of approximately 26 umoles/kg. A piperidine derivative possessing NK-3 receptor antagonist activity ((+)-(R)-3-[3-(4-Phenyl-4-N-methylacetamidopiperidin-1-yl)propyl)-3-(3,4-dichlorophenyl )-1-benzoylpiperidine) also reduced plasma testosterone of male dogs when given intravenously at a dose of approximately 26 umoles/kg. The single intravenous doses of (+)-(R)-3-[3-(4-Phenyl-4-N-methylacetamidopiperidin-1-yl)propyl]-3-(3,4-dichlorophenyl)-1-benzoylpiperidine and (S)-(-)-N-(α-Ethylbenzyl)-3-[3-[4-(2-methoxyphenyl)piperazin-1-yl]propoxy]-2-phenylquinoline-4-carboxamide that reduced plasma testosterone in male dogs had no effect on plasma estradiol concentration and did not lower plasma cortisol concentration.

Four daily oral doses ≥100 mg/kg (S)-(-)-N-(α-Ethylbenzyl)-3-(3-carboxy)propoxy-2-phenylquinoline-4-carboxamide or (S)-(-)-N-(α-Ethylbenzyl)-3-carboxymethoxy-2-phenylquinoline-4-carboxamide reduced the plasma testosterone concentration of male rats when measured at 4 hours after the last dose. Four daily oral doses of 300 mg/kg (S)-(-)-N-(α-Ethylbenzyl)-3-carboxymethoxy-2-phenylquinoline-4-carboxamide also reduced plasma LH concentration in male rats when measured at 4 hours after the last dose. Reductions in plasma testosterone also occurred in male rats following single oral doses of 30 to 1000 mg/kg (S)-(-)-N-(α-Ethylbenzyl)-3-carboxymethoxy-2-phenylquinoline-4-carboxamide. Stimulation of testosterone production *in vivo* by a LH agonist (human chorionic gonadotropin) was not blocked by a single oral dose of 1000 mg/kg (S)-(-)-N-(α-Ethylbenzyl)-3-carboxymethoxy-2-phenylquinoline-4-carboxamide in male rats.

*In vitro* studies utilizing primary cultures of Leydig cells isolated from the testes of dogs demonstrated that (S)-(-)-N-(α-Ethylbenzyl)-3-hydroxy-2-phenylquinoline-4-carboxamide and (+)-(R)-3-[3-(4-Phenyl-4-N-methylacetamidopiperidin-1-yl)propyl]-3-(3,4-dichlorophenyl)-1-benzoylpiperidine are capable of directly inhibiting testicular synthesis of testosterone in male dogs. This effect occurred at incubation concentrations between 10 and 100 uM. Furthermore, using lysed dog Leydig cells, it was demonstrated that the inhibition of testosterone synthesis by (S)-(-)-N-(α-Ethylbenzyl)-3-hydroxy-2-phenylquinoline-4-carboxamide involves inhibition of the enzyme (CYP17A1) in the testosterone synthetic pathway. (S)-(-)-N-(α-Ethylbenzyl)-3-carboxymethoxy-2-phenylquinoline-4-carboxamide did not inhibit testosterone synthesis in dog Leydig cells at the highest incubation concentration tested (100 uM). Neither (S)-(-)-N-(α-Ethylbenzyl)-3-carboxymethoxy-2-phenylquinoline-4-carboxamide nor (S)-(-)-N-(α-Ethylbenzyl)-3-(3-carboxy)propoxy-2-phenylquinoline-4-carboxamide inhibited testosterone synthesis in Leydig cells isolated from the testis of the rat at the highest incubation concentration tested (300 uM). In contrast, (+)-(R)-3-[3-(4-Phenyl-4-N-methylacetamidopiperidin-1-yl)propyl]-3-(3,4-dichlorophenyl)-1-benzoylpiperidine, and (S)-(-)-N-(α-Ethylbenzyl)-3-[(4-methylpiperazin-1-yl)methyl]-2-phenylquinoline-4-carboxamide inhibited testosterone synthesis of isolated rat Leydig cells at incubation concentrations between 3 and 300 uM.

Studies using isolated Leydig cells demonstrated that the NK-3 receptor antagonists (S)-(-)-N-(α-Ethylbenzyl)-3-hydroxy-2-phenylquinoline-4-carboxamide, (+)-(R)-3-[3-(4-Phenyl-4-N-methylacetamidopiperidin-1-yl)propyl]-3-(3,4-dichlorophenyl)-1-benzoylpiperidine, and (S)-(-)-N-(α-Ethylbenzyl)-3-[(4-methylpiperazin-1-yl)methyl]-2-phenylquinoline-4-carboxamide can directly inhibit testosterone synthesis in the testis. In contrast, the NK-3 receptor antagonist (S)-(-)-N-(α-Ethylbenzyl)-3-carboxymethoxy-2-phenylquinoline-4-carboxamide was not capable of inhibiting testosterone synthesis by Leydig cells *in vitro* or of blocking LH agonist-induced testosterone synthesis *in vivo.* However, this compound was capable of lowering both plasma LH and testosterone concentrations. Since testosterone synthesis in males requires stimulation by LH, the antiandrogenic activity of this compound appears to be due to its selective suppression of LH release. Finally, the NK-3 receptor antagonist (S)-(-)-N-(α-Ethylbenzyl)-3-hydroxy-2-phenylquinoline-4-carboxamide produced its antiandrogenic activity both by suppressing LH release and by directly inhibiting testosterone synthesis.

## Claims

1. Use of an NK-3 receptor antagonist selected from,
(S)-(-)-N-(α-Ethylbenzyl)-3-hydroxy-2-phenylquinoline-4-carboxamide;
(S)-(-)-N-(α-Ethylbenzyl)-3-[3-[4-(2-methoxyphenyl)piperazin-1-yl]propoxy]-2-phenylquinoline-4-carboxamide;
(+)-(R)-3-[3-(4-Phenyl-4-N-methylacetamidopiperidin-1-yl)propyl]-3-(3,4-dichlorophenyl)-1-benzoylpiperidine;
(S)-(-)-N-(α-Ethylbenzyl)-3-(3-carboxy)propoxy-2-phenylquinoline-4-carboxamide; and
(S)-(-)-N-(α-Ethylbenzyl)-3-carboxymethoxy-2-phenylquinoline-4-carboxamide; in the manufacture of a medicament for the treatment and/or prophylaxis of benign prostatic hyperplasia, metastatic prostatic carcinoma, testicular cancer, androgen dependent acne, male pattern baldness, precocious puberty in boys, hyperandrogenism, hirsutism, virilization, polycystic ovary syndrome, hyperandrogenism, insulin resistance and acanthosis nigricans syndrome, ovarian hyperthecosis, follicular maturation arrest, atresia, anovulation, dysmenorrhea, dysfunctional uterine bleeding, infertility and androgen-producing tumors.

2. Use according to claim 1, wherein the NK-3 receptor antagonist is (S)-(-)-N-(α-Ethylbenzyl)-3-hydroxy-2-phenylquinoline-4-carboxamide.

## Patentansprüche

1. Verwendung eines NK-3-Rezeptorantagonisten ausgewählt aus
(S)-(-)-N-(α-Ethylbenzyl)-3-hydroxy-2-phenylquinolin-4-carboxamid;
(S)-(-)-N-(α-Ethylbenzyl)-3-[3-[4-(2-methoxyphenyl)piperazin-1-yl]propoxy]-2-phenylquinolin-4-carboxamid;
(+)-(R)-3-[3-(4-Phenyl-4-N-methylacetamidopiperidin-1-yl)propyl]-3-(3,4-dichlorphenyl)-1-benzoylpiperidin;
(S)-(-)-N-(α-Ethylbenzyl)-3-(3-carboxy)propoxy-2-phenylquinolin-4-carboxamid; und
(S)-(-)-N-(α-Ethylbenzyl)-3-carboxymethoxy-2-phenylquinolin-4-carboxamid;
zur Herstellung eines Medikaments zur Behandlung und/oder Vorbeugung von gutartiger Prostatahyperplasie, metastasierendem Prostatakarzinom, Hodenkrebs, Androgen abhängiger Akne, den Mann betreffende Glatzenbildung, vorzeitiger Pubertät bei Jungen, Hyperandrogenismus, Hirsutismus, Virilisierung, Stein-Leventhal-Syndrom, Hyperandrogenismus, Insulinresistenz und Acanthosis nigricans, Ovarialhyperthekosis, Follikelreifungshemmung, Atresia, fehlender Ovulation, Dysmenorrhoe, dysfunktioneller Uterusblutung, Unfruchtbarkeit und Androgen produzierender Tumore.

2. Verwendung gemäß Anspruch 1, wobei der NK3-Rezeptorantagonist (S)-(-)-N-(α-Ethylbenzyl)-3-hydroxy-2-phenylquinolin-4-carboxamid ist.

## Revendications

1. Utilisation d'un antagoniste du récepteur NK-3, choisi entre,
le (S)-(-)-N-(α-éthylbenzyl)-3-hydroxy-2-phénylquinoléine-4-carboxamide ;
le (S)-(-)-N-(α-éthylbenzyl)-3-[3-[4-(2-méthoxyphényl)pipérazine-1-yl]propoxy]-2-phénylquinoléine-4-carboxamide ;
la (+)-(R)-3-[3-(4-phényl-4-N-méthylacétamidopipéridine-1-yl)propyl]-3-(3,4-dichlorophényl)-1-benzoylpipéridine ;
le (S)-(-)-N-(α-éthylbenzyl)-3-(3-carboxy)propoxy-2-phényl-quinoléine-4-carboxamide ; et
le (S)-(-)-N-(α-éthylbenzyl)-3-carboxyméthoxy-2-phénylquinoléine-4-carboxamide ;
dans la production d'un médicament destiné au traitement et/ou à la prophylaxie de l'hyperplasie prostatique bénigne, du carcinome prostatique métastatique, du cancer des testicules, de l'acné sous la dépendance d'androgènes, de la calvitie hippocratique masculine, de la puberté précoce chez les garçons, de l'hyperandrogénisme, de l'hirsutisme, de la virilisation, du syndrome d'ovaire polykystique, de l'hyperandrogénisme, de la résistance à l'insuline et du syndrome d'acanthosis nigricans, de l'hyperthécose ovarienne, de l'arrêt de maturation folliculaire, de l'atrésie, de l'anovulation, de la dysménorrhée, de saignements utérins dysfonctionnels, de la stérilité et de tumeurs productrices d'androgènes.

2. Utilisation suivant la revendication 1, dans laquelle l'antagoniste du récepteur NK-3 est le (S)-(-)-N-(α-éthylbenzyl)-3-hydroxy-2-phénylquinoléine-4-carboxamide.
